# EUROPEAN PATENT SPECIFICATION

(11) **EP 2 845 540 B1**
(45) Date of publication and mention of the grant of the patent: **25.07.2018**
(21) Application number: 14185027.1
(22) Date of filing: 21.02.2013
(51) Int. Cl.: A61B 6/00

(54) **RADIOGRAPHIC IMAGE CAPTURING DEVICE**
RÖNTGENBILDERFASSUNGSVORRICHTUNG
DISPOSITIF DE CAPTURE D'IMAGES RADIOGRAPHIQUES

(30) Priority: 28.02.2012 JP 2012040815
(43) Date of publication of application: 11.03.2015
(62) Divisional of application: 13156096.3
(73) Proprietor: Konica Minolta Medical & Graphic, Inc., Hino-shi, Tokyo 191-8511 (JP)
(72) Inventor: NISHIJIMA, Yuuichi, Tokyo 1918511 (JP); SUZUKI, Kenichirou, Tokyo 191-8511 (JP); TAKAGI, Tatsuya, Tokyo 1918511 (JP)
(74) Representative: Higgs, Jonathan

(56) References cited:
- EP-A1- 2 277 444
- DE-A1-102006 033 716
- US-A1- 2005 243 967
- US-A1- 2009 189 761

## Description

The present invention relates to a radiographic image capturing system.

For the purpose of diagnosing diseases, radiographic images captured using radiations represented by X-ray images are widely used. Such medical radiographic images are conventionally captured by using screen films. However, recently, CR (Computed Radiography) apparatus including a stimulable phosphor plate (also called a stimulable phosphor sheet) is widely used in order to digitalize the radiographic images.

In such CR apparatuses, radiation which transmitted through a subject such as the body of a patient, the radiation being emitted from a predetermined radiation source, is received by the stimulable phosphor plate and radiation energy holding the subject information is accumulated therein. Further, after radiographic image capturing is finished, the stimulable phosphor plate is scanned with excitation light such as a laser beam or the like and thereby, photoelectric conversion is performed on the accelerated phosphorescent light emitted from the stimulable phosphor plate to be read as image data.

As for the CR apparatus, there is known a CR cassette where the above stimulable phosphor plate is housed in a casing. In a case where image capturing is performed by using the CR cassette, the CR cassette is to be inserted in a reading device after the image capturing and reading processing to read image data by taking the stimulabel phosphor plate out from the CR cassette is performed in the reading device.

Further, there is a CR apparatus in which the above described stimulable phosphor plate and the reading device are integrally formed. In such case, when radiation is emitted to the stimulable phosphor plate through a subject, image data is to be read automatically with the reading device by the stimulable phosphor plate automatically moving in the apparatus.

On the other hand, in recent years, there is developed a radiographic image capturing apparatus which detects the emitted radiation with radiation detection elements and obtains as image data, the radiation detection elements such as photodiodes or the like being arranged on a substrate in a two dimensional manner (in matrix), as an apparatus in place for the CR apparatus. This type of radiographic image capturing apparatus is known as FPD (Flat Panel Detector).

Conventionally, the radiographic image capturing apparatus was developed as a so-called dedicated type (also called fixed type or the like) which is integrally formed with a supporting platform (for example, see Japanese Patent No. 3890163 and Japanese Published Unexamined Application No. H9-73144) and not portable. Recently, there is developed a radiographic image capturing apparatus which is portable where the radiation detection elements and the like are housed in a casing and has been put to practical use (fore example, see Japanese Published Unexamined Application No. 2006-058124 and Japanese Published Unexamined Application No. H6-342099).

Here, as for such radiographic image capturing apparatus, there is known a so-called direct type radiographic image capturing apparatus which generates charges with detection elements according to the dose of radiation such as X-ray emitted to convert into electric signals and a so-called indirect type radiographic image capturing apparatus which converts the emitted radiation into electromagnetic waves of other wavelength such as visible light with a scintillator or the like and thereafter, generates charges with photoelectric conversion elements such as photodiodes or the like according to the energy of the electromagnetic waves which are converted and emitted to convert into electric signals. Further, in the present invention, the detection elements in the direct type radiographic image capturing apparatus and the photoelectric conversion elements in the indirect type radiographic image capturing apparatus are called radiation detection elements all together.

Moreover, in the present invention, the dedicated type and the portable type radiographic image capturing apparatuses are called the FPD apparatus all together. Specifically, there is a case where the portable radiographic image capturing apparatus is called the FPD cassette. Further, the method of performing radiographic image capturing by using the FPD apparatus including a FPD cassette is shortened and called the FPD method and the method of performing radiographic image capturing by using the CR apparatus including the CR cassette is shortened and called the CR method.

In radiographic image capturing using the CR apparatus, an operator such as a technician can perform image capturing by emitting radiation at his (her) arbitrary timing, that is, at the timing the operator wishes to emit, to the CR apparatus. On the other hand, the FPD apparatus will not be in condition for receiving radiation unless the necessary processing such as reset processing of each radiation detection element for removing the charge remaining in each radiation detection element and so-called dark charge (also called dark current) which generates in each element is finished.

Therefore, differently from the CR apparatus, the FPD apparatus has a characteristic that an operator cannot perform image capturing by emitting radiation to the FPD apparatus unless the FPD apparatus is in a state for performing radiographic image capturing.

Moreover, for the reason that the FPD cassette is much more expensive comparing to the CR cassette and the like, at this time, it is impossible for all of the facilities such as hospitals and clinics where the CR apparatuses are already installed to replace their CR apparatuses with the FPD apparatuses at once. It is considered that some of the CR apparatuses which are already installed in the facility and the newly installed FPD apparatuses are to be used in a mixed manner at least for a while.

In such case, there are some aspects that controlling needs to be different in the FPD apparatus and in the CR apparatus. However, for example, International Publication No. 2010/032494, Japanese Unexamined Patent Application Publication No. 2011-502699 and the like describe radiographic image capturing systems which can appropriately switch the controlling. Document DE 102006033716 represents the closest prior art disclosing the preamble of independent claim 1. In the above mentioned conventional radiographic image capturing systems, because the FPD apparatus is to be installed in a facility in which the CR apparatus is already installed, the controlling method for CR apparatus (that is, controlling method for allowing emission at an arbitrary timing) is normally set in the default sate of the system due to the consideration that maintaining the work flow of an operator such as a technician established in the facility needs to be prioritized.

That is, in the initial state when the radiographic image capturing system is activated, the activation is carried out so that the controlling method of each device in the system is to be the controlling method for CR apparatus. When image capturing using FPD apparatus is to be performed, the controlling method is to be switched to the controlling method for FPD apparatus for the first time an operator such as a technician performing a predetermined operation (that is, a synchronous method where a signal or the like is sent and received between a radiation emission device and the FPD apparatus to confirm synchronism with each other in advance).

However, when the controlling method for CR apparatus is set in the default state (initial state) of the radiographic image capturing system, an operator such as a technician can emit radiation at his (her) arbitrary timing as described above. Therefore, even when the FPD apparatus is used in radiographic image capturing, that is, when image capturing is to be performed in the FPD method, radiation can be emitted at his (her) arbitrary timing without confirmation of synchronism. In addition, radiation can be emitted during initial activation of the system.

That is, despite an operator thinking that radiation will not be emitted immediately even when the after-mentioned exposure switch is operated by being controlled by the synchronous method because the FPD apparatus is to be used in the image capturing, radiation could be emitted immediately when the exposure switch is operated.

In view of the above, in a condition where both the FPD apparatus and the CR apparatus exist together, it is needed to configure so that an operator such as a technician cannot carelessly allows radiation emission.

Further, unless the controlling method for CR apparatus and the controlling method for FPD apparatus are switched appropriately when the image capturing method is switched to the FPD method from the CR method or to the CR method from the FPD method, recapturing may be necessary due to the radiographic image capturing not being performed properly.

Therefore, it is needed to configure so that the controlling method of each device can be switched appropriately when the image capturing method is switched in the case of the above configuration.

The present invention, which is defined by independent claim 1, is made in view of the above problems, and an object of the present invention is to provide a radiographic image capturing system which can appropriately prevent an operator from carelessly emitting radiation and which can appropriately switch the controlling method when the image capturing method is switched in a condition where the CR apparatus and the FPD apparatus exist together.

In order to solve the above problems, according to one aspect of the preferred embodiment of the present invention, there is provided a radiographic image capturing system including a FPD apparatus which reads a charge generated in each of a plurality of radiation detection elements due to radiation emission as image data, the plurality of radiation detection elements being arranged in a two dimensional manner, a CR apparatus which accumulates energy of radiation which is emitted in a stimulable phosphor plate, the stimulable phosphor plate being embedded in the CR apparatus, a radiation emission device which includes a radiation source which emits the radiation to the FPD apparatus or the CR apparatus through a subject, an exposure switch which instructs the radiation emission device to start the radiation emission, and a console which selects an image capturing order information in which at least an image capturing body part and an image capturing condition are set and which makes the image data read out from the FPD apparatus or the CR apparatus in a radiographic image capturing based on the selected image capturing order information be associated with the image capturing order information, and the console sends a first signal indicating that the radiographic image capturing is to be performed by using the CR apparatus to the radiation emission device when the piece of image capturing order information which indicates that the radiographic image capturing is to be performed by using the CR apparatus is selected, and when the radiation emission device receives an instruction for starting the radiation emission from the exposure switch in a state where the first signal is not received from the console, the radiation emission device makes the radiation source emit the radiation after an interlock release signal is received directly from the FPD apparatus or via the console, the FPD apparatus showing a ready condition for receiving the radiation emission, and when the radiation emission device receives the instruction for starting the radiation emission from the exposure switch after the first signal is received from the console, the radiation emission device makes the radiation source emit the radiation immediately in response to the instruction.

The present invention will become more fully understood from the detailed description given hereinbelow and the appended drawings which are given by way of illustration only, and thus are not intended as a definition of the limits of the present invention, and wherein:
FIG. 1 is a diagram showing the entire configuration of a radiographic image capturing system according to each embodiment;
FIG. 2 is a sectional view of a FPD cassette according to the embodiments;
FIG. 3 is a plane view showing a configuration of a substrate of the FPD cassette according to the embodiments;
FIG. 4 is a schematic sectional view showing a state where a connector of the FPD which is inserted in a cradle and a connector of the cradle are connected with each other;
FIG. 5 is a timing chart showing a processing flow until radiation is to be emitted to the FPD apparatus from a radiation source by an emission start signal being sent;
FIG. 6 is a diagram showing an example of image capturing order information;
FIG. 7 is a diagram showing an example of a selection screen which displays the image capturing order information;
FIG. 8 is a diagram showing an example of a screen which displays icons and the like corresponding respectively to the plurality of pieces of image capturing order information;
FIG. 9 is a diagram showing that a preview image is to be displayed at the position of icon I2 in the screen shown in FIG. 8;
FIG. 10 is a diagram showing a state where focus-display is switched to icon I3 from the state shown in FIG. 9;
FIG. 11 is a diagram showing a state where focus-display is further switched to icon I1 from the state shown in FIG. 10;
FIG. 12 is a diagram showing an example of a display indicating that radiographic image capturing is to be performed by using CR apparatus which is to be displayed in the icon I1 section;
FIG. 13 is a schematic view showing an example of outer appearance of an area exposure product meter;
FIG. 14 is a diagram showing the entire configuration of the radiographic image capturing system including a plurality of image capturing rooms;
FIG. 15 is a diagram showing an example of screen in which a tab is provided for each image capturing apparatus;
FIG. 16 is a diagram showing items and the like for QC test when complying with AAPM standard;
FIG. 17 is a diagram showing an example of QC phantom as QC tool; and
FIG. 18 is a diagram showing an example where analysis results of QC phantom image capturing data are displayed in the screen shown in FIG. 15.

Hereinafter, an embodiment of the radiographic image capturing system according to the present invention will be described with reference to the drawings. However, the present invention is not limited to the illustrated examples shown hereinafter.

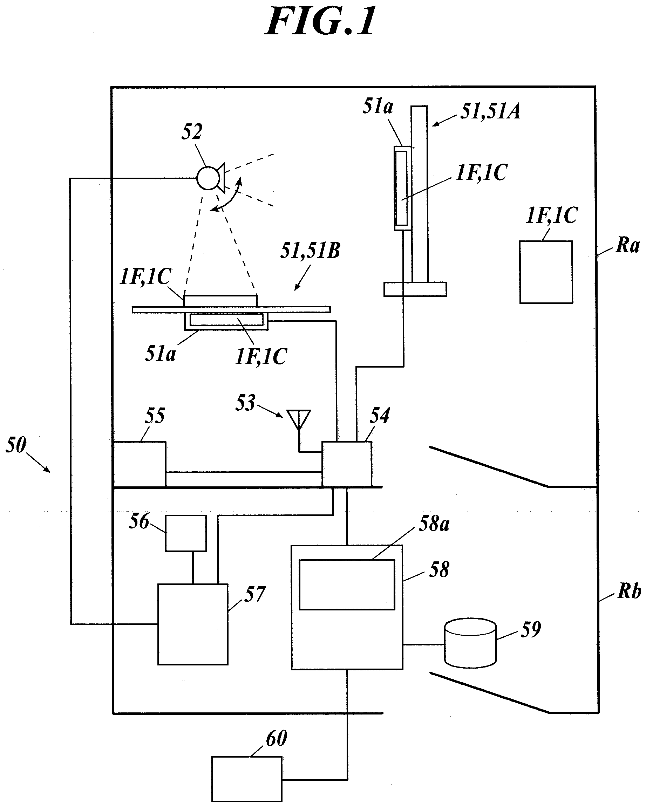

In the embodiment, a case where a FPD cassette 1F is provided as the FPD apparatus and a CR cassette 1C is provided as the CR apparatus and the FPD cassette 1F and the CR cassette 1C are respectively (alternatively) loaded in the cassette holding unit 51a of the bucky 51 to be used as shown in FIG. 1 will be described. However, the present invention can also be applied to a system where a dedicated type radiographic image capturing device is used as the FPD apparatus and the CR apparatus in which a stimulable phosphor plate and a reading device are integrally formed is used as the CR apparatus or to a system including the both of the dedicated type apparatuses, the FPD cassette 1F and the CR cassette 1C.

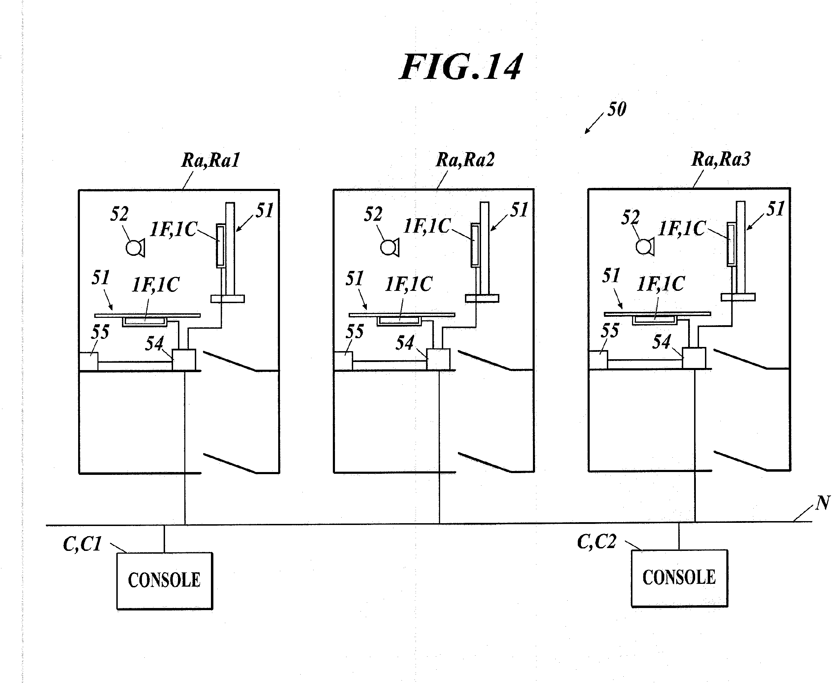

Further, in the embodiment, a case where an image capturing room Ra and a console 58 are associated to each other on one on one basis (1:1) will be described. However, the present invention can be applied to a system where a plurality of image capturing rooms Ra and a plurality of consoles 58 are connected via a network (m:n), for example, as shown in the after-mentioned FIG. 14.

First, general configuration of the radiographic image capturing system 50 according to the embodiment will be described. FIG. 1 is a diagram showing the entire configuration of the radiographic image capturing system according to the embodiment. The image capturing room Ra is a room where radiographic image capturing is performed by emitting radiation to a subject (that is, the image capturing body part of a patient) which is a part of the body of a patient, and a radiation source 52 or the like of the radiation emission device 57 for emitting radiation to the subject is disposed in the image capturing room Ra. The image capturing room Ra is shielded with lead or the like so that the radiation does not leak outside the image capturing room.

In the image capturing room Ra shown in FIG. 1, a case where the after-mentioned FPD cassette 1F and CR cassette 1C are used as the FPD apparatus and the CR apparatus by being loaded in the cassette holding unit 51a of the bucky 51 respectively is shown. The bucky 51, the radiation source 52 and the like will be described later.

First, the CR cassette 1C which is used for radiographic image capturing performed in the radiographic image capturing system 50 will be described.

As for the configuration of the CR cassette 1C, the configuration described in JP 2003-287833, for example, can be applied. As for the CR cassette 1C, a well-known CR cassette can be used and it is not specifically limited as long as it is a CR cassette which includes a stimulable phosphor plate in which energy of the emitted radiation is accumulated.

Further, in the embodiment, a barcode (not shown in the drawing) which holds identification information and the like of the CR cassette 1C is adhered on the back side or the like of the CR cassette 1C, for example, and as described later, when image data D is to be read out from the stimulable phosphor plate by loading the CR cassette 1C in the after-mentioned image reading device 60 which is disposed outside the image capturing room Ra, for example, after image capturing, the identification information and the like of the CR cassette 1C are read from the barcode by the image reading device 60.

Then, the image data D which is read by the image reading device 60 is sent to the consoled 58 (see FIG. 1) in a state where the identification information and the like of the CR cassette 1C is attached to the image data D. When a CR apparatus in which a stimulable phosphor plate and a reading device are integrally formed is used as the CR apparatus, it is needless to say that an image reading device 60 does not need to be provided separately from the CR apparatus because the image data D and the identification information and the like are sent to the console 58 from the CR apparatus.

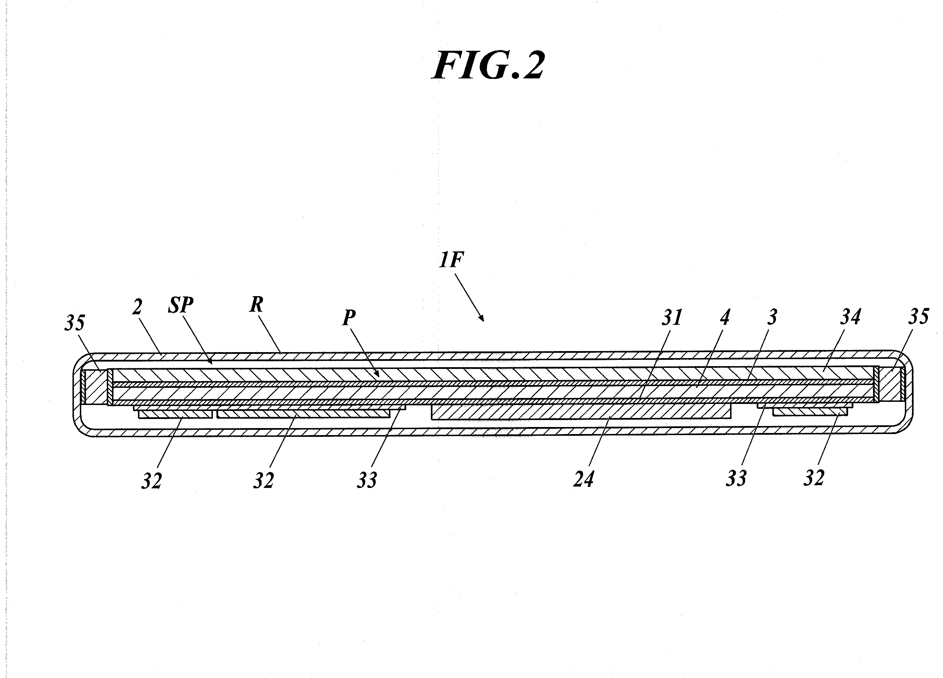

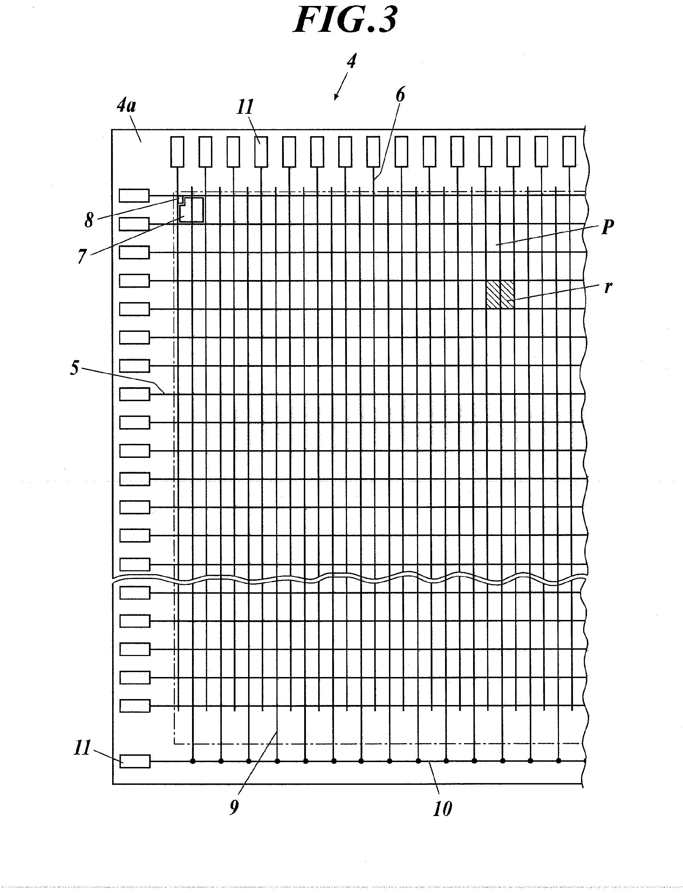

Next, the FPD cassette 1F used in the radiographic image capturing performed in the radiographic image capturing system 50 will be described. FIG. 2 is a sectional diagram of the FPD cassette according to the embodiment and FIG. 3 is a plane view showing the configuration of the substrate of the FPD cassette.

Hereinafter, so-called indirect type FPD cassette which includes a scintillator and which converts the emitted radiations into radio waves of other wave length such as visible light and obtains electric signals will be described as the FPD cassette 1F. However, the present invention can also be applied to a direct type FPD cassette which directly detects radiation with radiation detection elements without a scintillator.

In the embodiment, the FPD cassette 1F is configured so as to house a sensor panel SP which includes the scintillator 3, the substrate 4 and the like in a casing 2 made of carbon as shown in FIG. 2, wherein the casing 2 has a radiation entering surface R which is the surface subjected to radiation. Further, although it is omitted in FIG. 2, an antenna device which is the communication device for sending image data D and the like to the after-mentioned console 58 (see FIG. 1) in a wireless manner is provided in the casing 2.

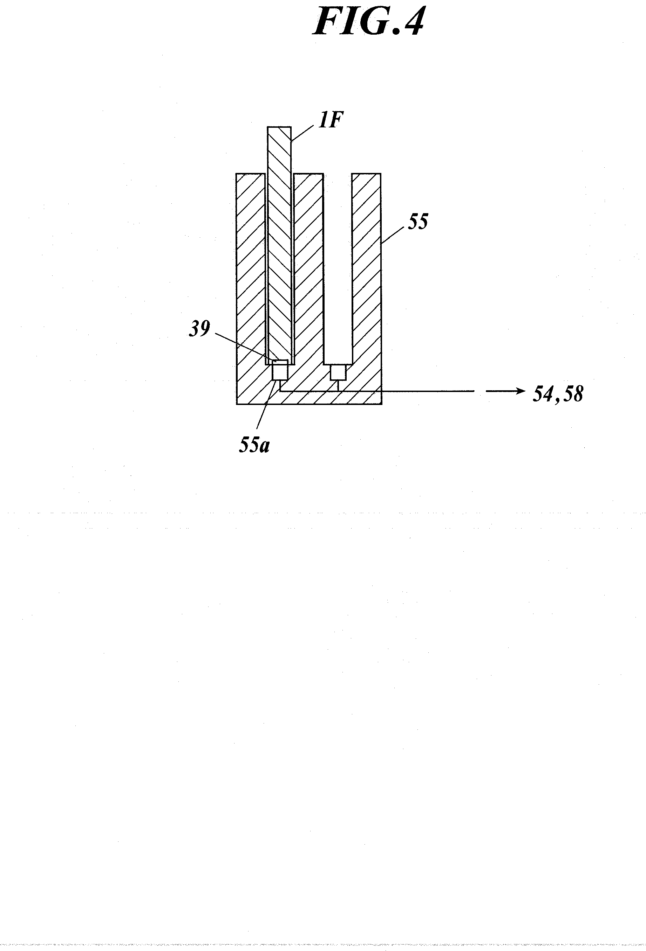

Moreover, although it is omitted in FIG. 2, the FPD cassette 1F includes a connector 39 (see after-mentioned FIG. 4) on a side surface of the casing 2 or the like in the embodiment, and signals, data and the like may be sent to the console 58 and the like via the connector 39 in a wired manner. Therefore, the connector 39 also functions as the communication device of the FPD cassette 1F.

As shown in FIG. 2, a base 31 is disposed in the casing 2 and the substrate 4 is provided on the radiation entering surface side R (hereinafter, simply called the upper surface side) of the base 31 via a thin plate of lead or such like (not shown in the drawing). Further, the scintillator 3 which converts the emitted radiation into light such as visible light is provided on the scintillator substrate 34 and the scintillator 3 is adhered thereto so as to face the substrate 4.

On the lower surface of the base 31, PCB board 33 on which electronic parts 32 and the like are disposed, a battery 24 and the like are attached. In such way, a sensor panel SP is formed with the base 31, the substrate 4 and the like. Further, in the embodiment, buffer materials 35 are provided between the sides of the casing 2 and the sensor panel SP.

As shown in FIG. 3, a plurality of scanning lines 5 and a plurality of signal lines 6 are arranged so as to cross each other on the upper surface 4a (that is, the side of the substrate 4 that faces the scintillator 3 in FIG. 2) of the substrate 4. In each of the small regions r which are sectioned by the plurality of scanning lines 5 and the plurality of signal lines 6 on the surface 4a of the substrate 4, a radiation detection element 7 is provided.

In such way, the entire small regions r where a plurality of radiation detection elements 7 are provided in two dimensional manner wherein the small regions r which are sectioned by the scanning lines 5 and the signal lines 6, that is, the region indicated by dashed lines in FIG. 3 is the detection unit P of the FPD cassette 1F. Although photodiodes are used as the radiation detection elements 7 in the embodiment, phototransistors or the like may also be used, for example.

In each radiation detection element 7, a thin film transistor 8 (hereinafter called TFT) is provided as a switch unit. Each TFT 8 makes the charge generated and accumulated in each radiation detection element 7 due to radiation be released to a signal line 6 when ON voltage is applied to the TFT 8 through a scanning line 5.

Although it is not shown in the drawing, a reading circuit which includes an amplifier circuit and the like is connected to each signal line 6 via an input/output terminal 11, and each reading circuit converts the charges released from each radiation detection element 7 into image data D to read out the data in compliance with an instruction from the control unit of the FPD cassette 1F. The read image data D is stored in a storage unit (not shown in the drawing) of the FPD cassette 1F.

Then, in the embodiment, the FPD cassette 1F thins the read image data D at a predetermined rate, creates preview data Dp from the image data D and sends the preview data Dp to the console 58. When the console 58 receives the preview data Dp from the FPD cassette 1F, the console 58 makes a display unit 58a display a preview image p_pre on the basis of the preview data Dp. This will be described later.

When the FPD cassette 1F sends the preview data Dp as described above, the remaining image data D stored in the storage unit is sent to the console 58 via the above described communication unit at a predetermined timing or in response to a send request from the console 58.

Further, in the FPD cassette 1F, offset data O as an offset due to so-called dark charge (also called dark current), the offset data O being superimposed on the image data D, is read out at a predetermined timing. Further, the read offset data O is stored in the storage unit and is also sent to the console 58 with the remaining image data D.

In the embodiment, the console 58 also functions as an image processing device. When image data D and offset data O are sent to the console 58 from the FPD cassette 1F, the console 58 performs a detailed image processing including offset correction, gain correction, bad pixel correction, tone correction according to image capturing body part and the like and generates a final radiographic image p (that is, radiographic image which is also called diagnosis-providing image) on the basis of the image data D and offset data O.

As described above, also in a case where image data D which is captured with the CR cassette 1C and read out in the image reading device 60 and image data D which is read out in the CR apparatus in which a stimulable phosphor plate and a reading device are integrally formed are sent, the console 58 similarly performs a detailed image processing and generates a radiographic image p.

Next, other devices and the like in the radiographic image capturing system 50 will be described.

As shown in FIG. 1, the bucky 51 can be used by loading the FPD cassette 1F or the CR cassette 1C in the cassette holding unit 51 (also called a holder or such like). Here, FIG. 1 shows a case where the bucky 51A for standing position image capturing and the bucky 51B for recumbent position image capturing are provided as the bucky 51. However, the present invention can be applied to cases where only the bucky 51A for standing position image capturing is provided and where only the bucky 51B for recumbent position image capturing is provided.

In the embodiment, the bucky 51 is configured so that it can also be used by loading the CR cassette 1C which is same as a conventional CR cassette in the cassette holding unit 51a, and an existing bucky which is provided for the conventional CR cassette in the image capturing room Ra can be available continuously.

Therefore, in the embodiment, the FPD cassette 1F is formed so that its outer size is to be similar to that of the CR cassette 1C. That is, the CR cassette 1C is formed in the size of 14 inches × 17 inches or the like that complies with JIS standard size (corresponding international standard is IEC 60406) of conventional screen film cassette. Further, the CR cassette 1C is formed so that its thickness in radiation entering direction is within the range of 15mm+ 1mm to 15mm-2mm.

Therefore, in order to allow the FPD cassette 1F to be loaded and to be used in the bucky 51 in which the CR cassette 1C in the JIS standard size can be loaded, the FPD cassette 1F is also formed in the size that complies with the JIS standard of screen film cassette which the CR cassette 1C complies with.

In a case where the existing bucky for screen/film cassette and CR cassette which are compatible is not to be used, there is no need for forming the FPD cassette 1F in the above described size and the FPD cassette 1F can be formed in arbitrary size and shape.

In the embodiment, when the connector 39 (see the after-mentioned FIG. 4) of the bucky 51 and the connector (not shown in the drawing) of the FPD cassette 1F are connected, the bucky 51 reads out the cassette ID from the FPD cassette 1F, makes the cassette ID of the FPD cassette IF and the bucky ID be associated with each other and sends the cassette ID and the bucky ID to the console 58.

In a case where a CR cassette 1C is to be loaded in the bucky 51, the barcode of the CR cassette 1C is to be read by a reading device (not shown in the drawing) provided in the bucky 51. When the barcode of the CR cassette 1C is read by the reading device, the bucky 51 makes the identification information such as the cassette ID of the CR cassette 1C and the bucky ID which is the identification information of itself be associated with each other and sends the identification information of the CR cassette 1C and the bucky ID to the console 58 in the similar manner as described above.

The console 58 recognizes the cassette ID of the FPD cassette 1F or the CR cassette 1C sent from the bucky 51 in association with the bucky ID of the bucky 51 and manages which cassette is loaded in which bucky 51.

As shown in FIG. 1, at least one radiation source 52 which emits radiation to a subject is provided in the image capturing room Ra. The radiation source 52 is disposed by being hung from the ceiling of the image capturing room Ra, for example, and the radiation source 52 is activated on the basis of an instruction from the console 58 at the time of image capturing and is moved to a predetermined position by a moving unit (not shown in the drawing).

In the embodiment, radiation can be emitted to the FPD cassette 1F or the CR cassette 1C which is loaded in the bucky 51A for standing position image capturing or bucky 51B for recumbent position image capturing by changing the direction of radiation emission.

Further, the FPD cassette 1F and the CR cassette 1C can be used for image capturing in a state not being loaded in the bucky 51. In a state where the FPD cassette 1F or the CR cassette 1C is held against a body part directly or where the FPD cassette 1F or the CR cassette 1C is inserted between a table of the bucky 51B and the body of a patient, radiographic image capturing can be carried out by emitting radiation from the above described radiation source 52 or a portable radiation source for a preferred distance and in a preferred direction.

The radiation source 52 is provided with a X-ray tube. When a predetermined X-ray tube voltage, X-ray tube current or the like is supplied to the X-ray tube from the after-mentioned radiation emission device 57, the X-ray tube emits radiation in exposure dose according to the X-ray tube voltage or the like for the set irradiation time period.

On the other hand, as described above, signals and data cannot be sent or received via the antenna device from the FPD cassette 1F in the image capturing room Ra because the image capturing room Ra is shielded with lead or the like. Therefore, as showing in FIG. 1, a relay device 54 (also called a base station) provided with an access point 53 which relays the communication when the FPD cassette 1F and the console 58 and the like communicate with each other is provided.

Moreover, the relay device 54 is connected with the radiation emission device 57 and the cradle 55 in addition to the bucky 51 and the console 58. In the relay device 54, a converter (not shown in the drawing) which performs conversion of signals for LAN (Local Area Network) communication sent to the radiation emission device 57 from the radiographic image capturing device 1, the console 58 and the like into signals for radiation emission device 57 and the inverse conversion of the above is embedded.

The cradle 55 is connected to the relay device 54. As shown in FIG. 4, the FPD cassette 1F is to be inserted in the cradle 55 when the FPD cassette 1F is brought into the image capturing room Ra. When the FPD cassette 1F is inserted in the cradle 55 and the connector 39 of the FPD cassette 1F and the connector 55a of the cradle 55 are connected, the cassette ID is to be notified from the FPD cassette 1F to the relay device 54 via the cradle 55.

When the cassette ID of the FPD cassette 1F is sent to the relay device 54 from the cradle 55, the relay device 54 notifies the cassette ID to the console 58. In such way, the console 58 manages that the FPD cassette 1F is brought into the image capturing room Ra.

As described above, in a case where a plurality of image capturing rooms Ra are connected with the consoles 58 via a network, for example, the cradle 55 can be provided in each image capturing room Ra and identification information of each image capturing room Ra and identification information of the cradle 55 can be stored in the console 58 so as to be associated with each other.

By configuring so as to also send the identification information of the cradle 55 when the cassette ID is to be sent to the console 58 from the FPD cassette 1F which is inserted in the cradle 55, to which image capturing room Ra the FPD cassette 1F was brought in can be managed. Here, instead of the console 58 performing the management as described above, configuration may be such that a management server or the like separate from the console 58 performs the management.

Normally, the cradle 55 is used for holding and charging the FPD cassette 1F and the like, and configuration may be such that the cradle 55 also includes the function of charging and such like in the embodiment. Further, the cradle 55 can be set in either of the image capturing room Ra and the front room Rb, and when the cradle 55 is to be set in the image capturing room Ra, the cradle 55 is set at a position where radiation emitted from the radiation source 52 does not reach, that is, at a position in a corner of the image capturing room Ra or such like, for example.

As shown in FIG. 1, the radiation emission device 57 which includes the exposure switch 56 to give the radiation source 52 an instruction to start radiation emission and the like is provided in the front room Rb (also called an operation room). It is configured that radiation is to be emitted from the radiation source 52 when the exposure switch 56 is operated twice by an operator such as a technician, for example.

That is, when an operator carries out a first stage operation (for example, pushing the button halfway), the exposure switch 56 sends an activation signal to the radiation emission device 57. Then, when the radiation emission device 57 receives the activation signal, the radiation emission device 57 makes rotation of positive pole of the X-ray tube of the radiation source 52 start or such like to make the radiation source 52 be in standby state. Thereafter, when an operator carries out a second stage operation (for example, fully pushing the button), an emission start signal is to be sent to the radiation emission device 57 from the exposure switch 56.

The radiation emission device 57 is configured so as to switch the way how radiation is to be emitted between a case where the CR cassette 1C is loaded in the bucky 51 and a case where the FPD cassette 1F is loaded in the bucky 51. That is, the radiation emission device 57 switches the way how radiation is to be emitted between a case where radiographic image capturing is performed by using CR apparatus and a case where radiographic image capturing is performed by using FPD apparatus.

In particular, in a case where radiographic image capturing is performed by using the CR apparatus (that is, the CR cassette 1C and the CR apparatus wherein a stimulable phosphor plate and a reading device are integrally formed, for example), when an emission start signal is sent from the exposure switch 56 in which the second stage operation is carried out as described above, the radiation emission device 57 allows radiation to be emitted to the CR apparatus from the radiation source 52 immediately in response to the emission start signal.

On the other hand, in a case where radiographic image capturing is performed by using the FPD apparatus (that is, the FPD cassette 1F and the dedicated-type radiographic image capturing apparatus), when an emission start signal is sent from the exposure switch 56 in which the second stage operation is carried out as described above, the radiation emission device 57 sends the emission start signal to the FPD apparatus directly through the relay device 54 or further via the console 58.

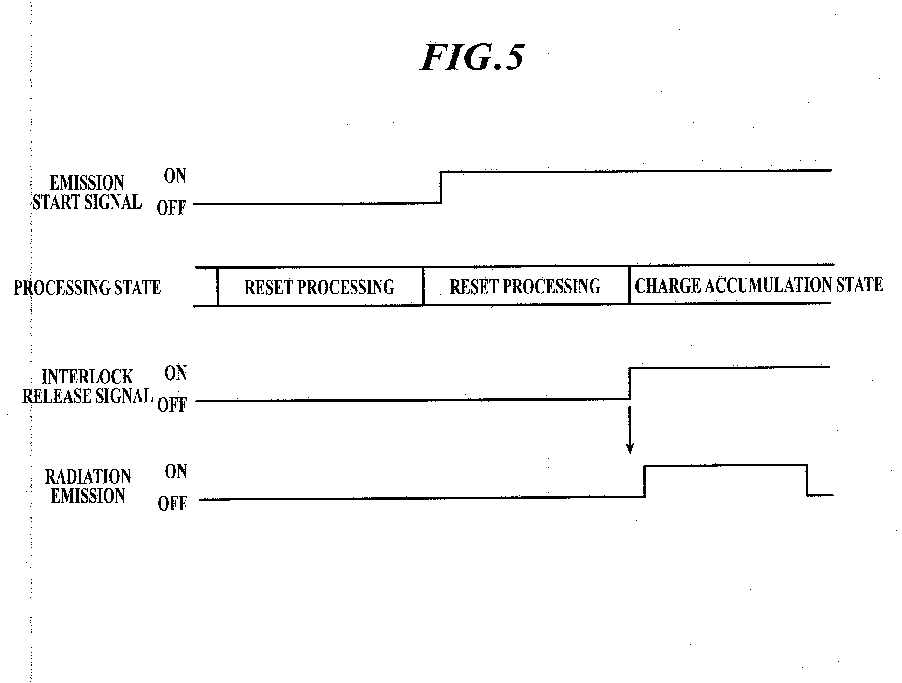

Thereafter, as shown in FIG. 5, when the radiation emission device 57 receives an interlock release signal from the FPD apparatus directly through the relay device 54 or further via the console 58, the FPD apparatus being in a condition for receiving radiation finished with necessary processing such as reset processing and the like of each radiation detection element 7 in preparation for image capturing, the radiation emission device 57 allows radiation to be emitted to the FPD apparatus from the radiation source 52 for the first time.

In such way, the radiation emission device 57 switches the way how radiation is to be emitted between the case where radiographic image capturing is performed by using the CR apparatus and the case where radiographic image capturing is performed by using the FPD apparatus. Here, how the switching is actually carried out is the essential matter of the present invention and will be described in detail later.

Here, instead of directly attaching the exposure switch 56 to the radiation emission device 57 as shown in FIG. 1, configuration may be such that the exposure switch 56 is connected to the relay device 54 and a signal from the exposure switch 56 is input to the radiation emission device 57 via the relay device 54.

The system shown in FIG. 1 is used when the existing radiation emission device 57 and exposure switch 56 (that is, the radiation emission device 57 and the exposure switch 56 are already installed for CR image capturing) are used as they are in a facility and a communication interface is released between the manufacturer of the radiation emission device and the manufacturer of the FPD apparatus so that the synchronous control flow can be installed additionally.

On the other hand, the system where the exposure switch 56 is connected to the relay device 54 described above is used in a case where the above mentioned communication interface is not released by modifying (that is, by relocating) the position of the existing exposure switch 56 or by nullifying the function of the existing exposure switch 56 and additionally installing a new exposure switch 56.

As shown in FIG. 1, in the embodiment, the console 58 is provided in the front room Rb. The consol 58 is composed of a computer or the like in which a CPU (Central Processing Unit), a ROM (Read Only Memory), a RAM (Random Access Memory), an input/output interface and the like (not shown in the drawing) are connected to a bus. Predetermined programs are stored in the ROM, and the console 58 reads out the needed program and expands the read program in the working region of the RAM to execute various types of processing in compliance with the program.

The display unit 58a composed of a CRT (Cathode Ray Tube), a LCD (Liquid Crystal Display) or the like is included in the console 58, and in addition, input units and the like such as a key board and a mouse (not shown in the drawing) are connected to the console 58. Further, a storage unit 59 composed of a hard disk or the like is built in the console 58 or is connected to the console 58.

Moreover, an image reading device 60 which reads out image data D from a stimulable phosphor plate of the CR cassette 1C is also connected to the console 58. As for the image reading device 60, the image reading device which is already installed in a facility such as the image reading device described in JP 2003-287833 or the like, for example, can be used. As for the image reading device 60, its configuration is not limited to the above as long as image data D can be read out from the stimulable phosphor plate in the CR cassette 1C.

Moreover, the console 58 sends signals and the like to the FPD cassette 1F, the bucky 51, the relay device 54, the cradle 55, the radiation emission device 57 and the image reading device 60 and obtains necessary information from the above units as needed and controls the operations of the devices and units in the radiographic image capturing system 50 and sets various types of conditions for the devices and units to control the entire system 50.

Further, although they are not shown in the drawings, HIS (Hospital Information System), RIS (Radiology Information System), PACS (Picture Archiving and Communication System) and the like are connected to the console 58 via a network or the like such as LAN.

Hereinafter, processing in the radiographic image capturing system 50 according to the above embodiment will be described following the procedure of radiographic image capturing.

First, an operator such as a technician inserts the FPD cassette 1F in the cradle 55 as described above when he or she brings the FPD cassette 1F into the image capturing room Ra (see FIG. 1). Then, the cassette ID of the FPD cassette 1F is notified to the console 58 from the FPD cassette 1F via the cradle 55 and the relay device 54. The console 58 recognizes that the FPD cassette 1F is brought into the image capturing room Ra and starts managing of the FPD cassette 1F.

Further, for example, when an operator loads the FPD cassette 1F in the cassette holding unit 51a of the bucky 51, the bucky 51 sends the Bucky ID and the cassette ID of the FPD cassette 1F to the console 58 by associating the IDs to each other as described above. Then, the console 58 recognized that the FPD cassette 1F is loaded in the bucky 51 and starts managing of the FPD cassette 1F.

Furthermore, for example, when an operator reads the barcode of the CR cassette 1C with the reading device of the bucky 51 and loads the CR cassette 1C in the cassette holding unit 51a of the bucky 51, the bucky 51 sends the bucky ID and the cassette ID of the CR cassette 1C to the console 58 by associating the IDs to each other as described above. Then, the console 58 recognizes that the CR cassette 1C is loaded in the bucky 51 and starts managing of the CR cassette 1C.

Subsequently, an operator moves to the console 58 and operates the input unit of the console 58 to obtain the image capturing order information relating to the radiographic image capturing to be performed on that day, for example, from HIS and RIS via network.

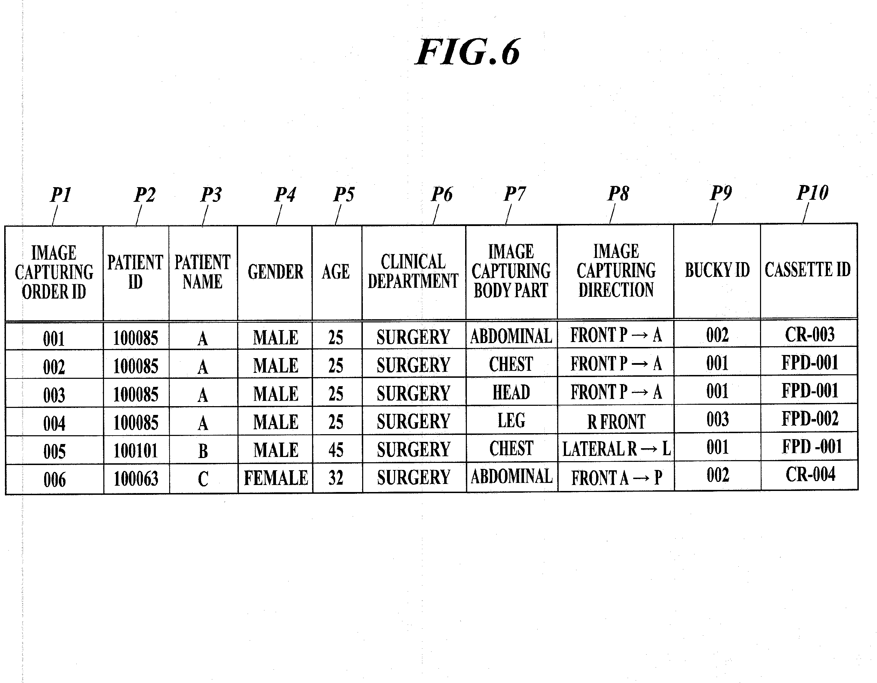

In the embodiment, each image capturing order information includes "patient ID" P2, "patient name" P3, "gender" P4, "age" P5 and "clinical department" P6 as patient information, "image capturing body part" P7 and "image capturing direction" P8 as image capturing conditions and such like as shown in FIG. 6, for example. Here, "image capturing order ID" P1 is to be automatically assigned to the image capturing order information in the order in which the image capturing order information was received.

In the embodiment, the image capturing order information further includes items such as "bucky ID" P9 which indicates the bucky 51 to be used and "cassette ID" P10 of the cassette to be used. In the example shown in FIG. 6, the bucky ID of "001" indicates the bucky 51A for standing position image capturing (see FIG. 1) and the bucky ID of "002" indicates the bucky 51B for recumbent position image capturing. Further, the bucky ID of "003" indicates that the cassette is to be used alone without being loaded in the bucky.

However, instead of specifying the above items (that is, which cassette is loaded in which bucky to perform image capturing) in the image capturing order information in advance as in the embodiment, radiographic image capturing can be performed automatically on the basis of the association between the bucky 51 and the cassette ID managed by the console 58 as described above.

In a case where the above items P9 and P10 are specified in advance in the image capturing order information as in the embodiment, when the association between the bucky 51 and the cassette ID which the console 58 manages and the association specified in advance in the image capturing order information are different, a warning sound is produced or a warning display is displayed in the display unit 58a of the console 58 and the cassette can be changed to the cassette specified in the image capturing order information or the association in the image capturing order information can be changed to the actual association.

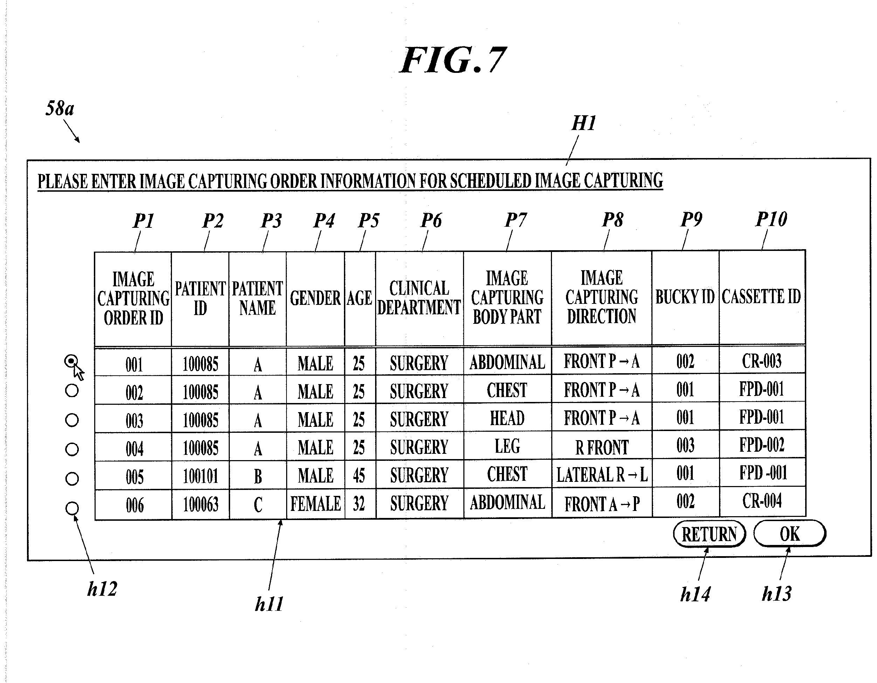

When the console 58 obtains the image capturing order information as described above, as shown in FIG. 7, a list of the image capturing order information is to be displayed in a list format on the selection screen H1 in the display unit 58a.

In the embodiment, an image capturing order information display section h11 for displaying the list of the image capturing order information is provided in the selection screen H1 and selection buttons h12 for selecting the image capturing order information which is scheduled to be performed are provided on the left side of the image capturing order information display section h11. An OK button h13 and a return button h14 are provided below the image capturing order information display section h11.

An operator selects one of the image capturing order information by clicking the selection button h12 and clicks the OK button h13. Here, configuration may be such that the order information is selected first and then the remaining image capturing order information relating to the same patient to whom the selected order information belongs to is automatically selected.

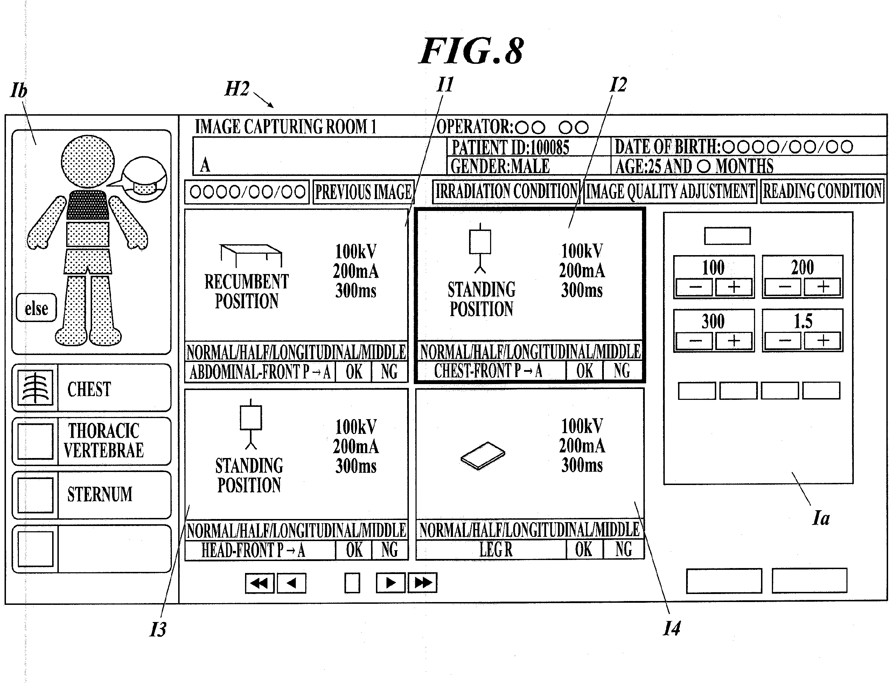

For example, when an operator selects four of the image capturing order information relating to a patient called "A" in the image capturing order information of FIG. 7 and clicks the OK button h13, the console 58 makes the display unit 58a display the screen H2 as shown in FIG. 8.

In the screen H2, the icons I corresponding to the four of the image capturing order information are displayed, and at the bottom of each icon I, a "NG" button icon which is to be clicked when the image needs to be recaptured after an operator reviewing the preview image p_pre displayed in the position of the icon I or when image processing to the radiographic image p needs to be redone and the like is displayed as described later.

Further, by clicking the "+" button or "-" button of each item in the display Ia for setting image capturing condition which is displayed in the right side in the screen H2, fine adjustment of the image capturing conditions such as X-ray tube voltage, X-ray tube current, irradiation time period and the like of the radiation source 52 of the radiation emission device 57 can be carried out to set the conditions.

In the embodiment, the console 58 selects any one icon I (the icon I2 in the case shown in FIG. 8) in the icons I1 to I4 displayed in the screen H2, and the selected icon I is displayed by being focused so as to be easily noticed. Then, image capturing based on the image capturing order information corresponding to the focused icon I is to be performed. Here, in a case where an operator wishes to perform image capturing based on another image capturing order information, an operator can move the focus by clicking and selecting another icon I corresponding to the image capturing order information.

Moreover, in the left side of the screen H2, the image capturing body part P7 (see FIGS. 6 and 7) specified in the image capturing order information corresponding to the icon I which is displayed by being focused is shown on a human body model Ib which is displayed so that an operator can recognize the image capturing body part P7 at a glance.

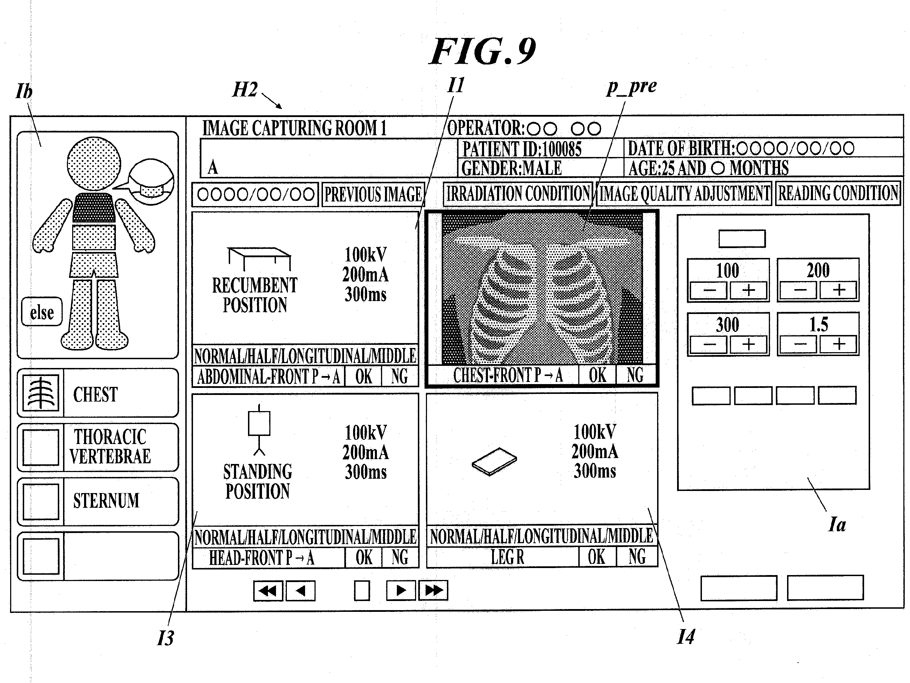

On the other hand, when radiographic image capturing based on the image capturing order information corresponding to the icon I displayed by being focused is performed, the preview data Dp created from the image data D of each radiation detection element 7 is sent to the console 58 from the FPD cassette 1F as described above. When the preview data Dp is sent from the FPD cassette 1F, the console 58 generates a preview image p_pre on the basis of the preview data Dp which is sent and displays the preview image p_pre at the position of the original icon I2 which was focused as shown in FIG. 9.

Here, the preview image p_pre can be enlarged and displayed in the screen H2 for a predetermined period of time so that it is easier for an operator to review the preview image p_pre.

When the image data D and the like are sent to the console 58 from the FPD cassette 1F, the console 58 refers to the cassette ID of the FPD cassette 1F attached to the image data D and the like to confirm that the FPD cassette 1F is the cassette specified in the image capturing order information corresponding to the icon I and associates the image data D and the like to the image capturing order information.

Then, if an operator who reviews the preview image p_pre does not click the "NG" button icon during the predetermined period of time the preview image p_pre is displayed, the console 58 switches the icon to be focus-displayed to the icon 13, for example, and starts generation processing of a radiographic image p for diagnosis based on the image data D and the like sent from the FPD cassette 1F in the image capturing corresponding to the original icon 12.

Here, if an operator who reviews the preview image p_pre clicks the "NG" button icon during the predetermined period of time the preview image p_pre is displayed, the association of the image data D and the like to the image capturing order information is canceled and the preview data Dp and the image data D are deleted. Further, generation processing of radiographic image p based on the image data D and the like is not performed.

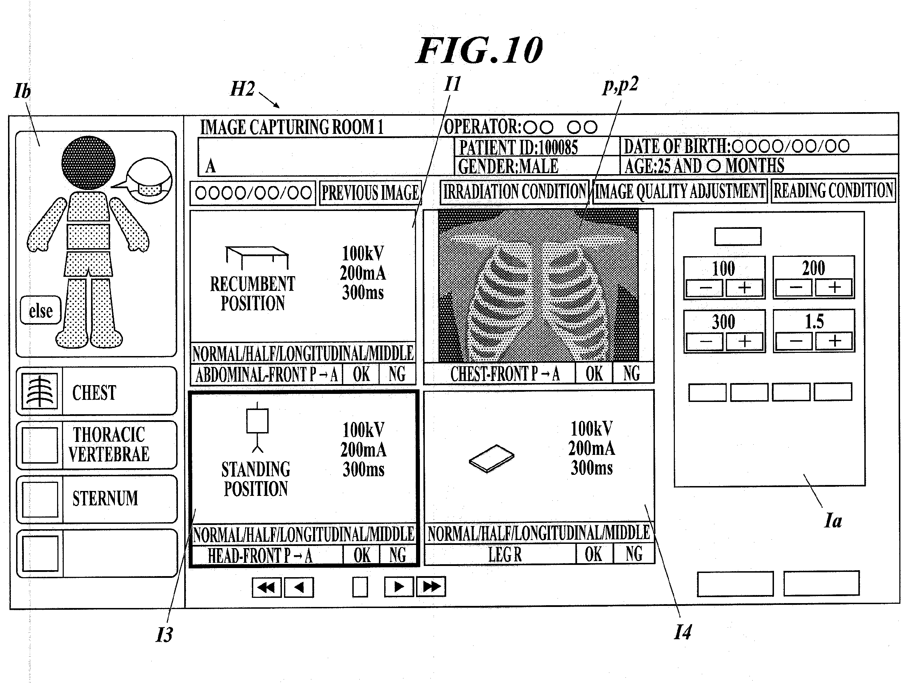

When the console 58 generates the radiographic image p for diagnosis, the radiographic image p is displayed at the position of the original icon I2 as shown in FIG. 10. Then, if an operator who reviewed the radiographic image p determines that the generated radiographic image p is good and clicks the "OK" button icon, the radiographic image p for diagnosis is confirmed and is made to be associated with the image capturing order information.

In the embodiment, when radiographic image capturing is performed by using the CR cassette 1C and when the image data D is sent to the console 58 from the image reading device 60 which read the image data D from the CR cassette 1C, the console 58 generates and displays the radiographic image p for diagnosis in the similar way as described above. Then, if an operator clicks the "OK" button icon, the console 58 confirms the radiographic image p for diagnosis and associates the radiographic image p with the image capturing order information.

On the other hand, as for the ways to focus-displaying the icons I1 to I4 and switching the focus-display among the icons I1 to I4 and the like in the screen H2 of the console 58, the method used in the system described in International publication No. 2011/142157 previously filed by the applicant of the present application is used. Please see International publication No. 2011/142157 for detail.

The feature thereof is that when a certain radiographic image capturing is finished, the console 58 switches the focus to the position of the icons I corresponding to the radiographic image capturing which can be performed in a state that requires minimum change in the conditions of the radiation source 52 and the like from the conditions at that time or in a sate that requires minimum preparation for the next image capturing for an operator such as a technician and minimum moving of a patient.

That is, for example, at the time when the image capturing of the image capturing order information corresponding to the icon I2 is finished as described above, the radiation source 52 is in a state where it can emit radiation to the bucky 51 for standing position image capturing to which a FPD cassette 1F is loaded. The image capturing of the front of the patient's head, that is, the image capturing of the image capturing order information corresponding to the icon I3 can be performed by the patient remaining standing near the bucky 51 for standing position image capturing, moving the FPD cassette 1F upward and slightly changing the direction of the radiation source 52 so as to face upward.

Therefore, in the case as described above, if an operator who reviews the preview image p_pre does not click the "NG" button icon during the predetermined period of time the preview image p_pre is displayed, the console 58 switches the icon to be focus-displayed to the icon I3 as shown in FIG. 10.

In such way, in the embodiment, the console 58 automatically switches between the icons I on the basis of the above criteria. Here, as described above, the focus-display can be switched by an operator clicking and selecting a different icon I corresponding to another image capturing order information when an operator disagrees with the focus-display of an icon I which is automatically switched by the console 58.

Next, configuration and the like characteristic to the present invention in the radiographic image capturing system 50 according to the embodiment will be described. Further, operation of the radiographic image capturing system 50 according to the embodiment will also be described.

As described above, in a radiographic image capturing system including both the FPD apparatus and the CR apparatus, the controlling method for CR apparatus is normally set in the default condition in the system because in many of such systems, the FPD apparatus is newly installed in a facility where the CR apparatus is already installed.

For example, when the icon I2 which corresponds to the image capturing order information that specifies to perform radiographic image capturing by using FPD apparatus including the FPD cassette 1F is selected and focus-displayed as described above, in response, the radiation source 52 is to be on standby and the FPD apparatus starts the needed processing such as reset processing of each radiation detection element 7 in preparation for image capturing.

However, in a conventional system, the radiation emission device 57 makes the radiation source 52 emit radiation immediately regardless of whether the FPD apparatus is in the condition for receiving radiation (that is, even during initialization after the power is turned on, for example) when an operator such as a technician operates the exposure switch 56 (see FIG. 1) because the controlling method for CR apparatus is set in the default state as described above.

In view of the above, in the present invention, the controlling method for FPD apparatus is to be set in the default state of the radiographic image capturing system 50. That is, even when an operator operates the exposure switch 56 in the default state, the radiation emission device 57 does not make the radiation source 52 emit radiation unless an interlock release signal is sent from the FPD apparatus.

Therefore, even when an operator such as a technician carelessly or unintentionally operates the exposure switch 56 and tries to emit radiation, radiation is prohibited until the needed processing such as reset processing of each radiation detection element 7 is finished in the FPD apparatus and the FPD apparatus is in the condition to receive radiation. Thus, the above problem can be prevented from occurring appropriately.

However, when the system is configured as described above, in a case where radiographic image capturing is to be performed by using CR apparatus, the radiation emission device 57 is to be in a state where it cannot make the radiation source 52 emit radiation forever even when an operator operates the exposure switch 56 because an interlock release signal is not to be sent from the CR apparatus.

In view of the above, the radiation emission device 57 needs to make the radiation source 52 emit radiation immediately when an operator operates the exposure switch 56 even when an interlock release signal is not sent by accurately (intentionally) switching the controlling method when radiographic image capturing is to be performed by using CR apparatus although the controlling method for FPD apparatus is set in the default state of the system.

In order to realize the above, in the embodiment, when the icon I corresponding to the image capturing order information which indicates to perform radiographic image capturing by using CR apparatus is selected, the console 58 sends a signal indicating that radiographic image capturing is to be performed by using CR apparatus to the radiation emitting apparatus 57.

If the radiation emission device 57 receives an emission start signal from the exposure switch 56 by an operator operating the exposure switch 56 after receiving the above signal from the console 58, the radiation emission device 57 immediately makes the radiation source 52 emit radiation according to the emission start signal because the radiographic image capturing is to be performed by using CR apparatus.

However, when the emission start signal is received from the exposure switch 56 by an operator operating the exposure switch 56 in a state where the above signal is not received from the console 58 (this state includes a state where image capturing using FPD apparatus is in progress, a sate where initial system activation is in progress, a state where system maintenance is in progress and such like), that is, in the default state, the radiation emission device 57 makes the radiation source 52 emit radiation when receiving the interlock release signal directly from the FPD apparatus which is now in the condition to receive radiation or via the console 58 because the radiographic image capturing is to be performed by using FPD apparatus.

Therefore, because the interlock release signal will not be sent from the FPD apparatus during activation or during system maintenance, other than during image capturing, unexpected exposure by an operator such as a technician or a service person can be prevented.

Specific description will be given hereinafter. For example, because the controlling method for FPD apparatus is set in the default state of the radiographic image capturing system 50, the radiation emission device 57 will not make the radiation source 52 emit radiation even if an operator such as a technician operates the exposure switch 56 in a state before a predetermined icon I is focus-displayed as shown in FIG. 8 in the display unit 58a of the console 58, that is, in a state before the selection screen H1 as shown in FIG.7 is displayed in the display unit 58a of the console 58 or a sate even before that, for example.

Further, when the icon I2 is focus-displayed in the screen H2 as shown in FIG. 9, for example, because the FPD cassette 1F having the cassette ID "FPD -001" is specified in the image capturing order information corresponding to the icon I2 (that is, the image capturing order information having the image capturing order ID "002" in FIG. 7), it is acceptable that the controlling method for FPD apparatus remains to be set as the controlling method.

Therefore, in such case, a signal indicating that radiographic image capturing is to be performed by using CR apparatus is not to be sent to the radiation emission device 57 from the console 58. Further, because the controlling mode remains as the mode for performing radiographic image capturing by using FPD apparatus, the radiation emission device 57 does not make the radiation source 52 emit radiation even when the emission start signal sent from the exposure switch 56 by an operator operating the exposure switch 56 is received unless the interlock release signal is received directly from the FPD apparatus or via the console 58.

Further, also in a case where the focus-display is switched to the icon I3 as the next radiographic image capturing as shown in FIG. 10, because the FPD cassette 1F having the cassette ID "FPD-001" is specified in the image capturing order information corresponding to the icon I3 (that is, the image capturing order information having the image capturing order ID "003" in FIG. 7), it is acceptable that the controlling method for FPD apparatus remains to be set as the controlling method. Therefore, the signal indicating that radiographic image capturing is to be performed by using CR apparatus is not to be sent to the radiation emission device 57 from the console 58 also in such case.

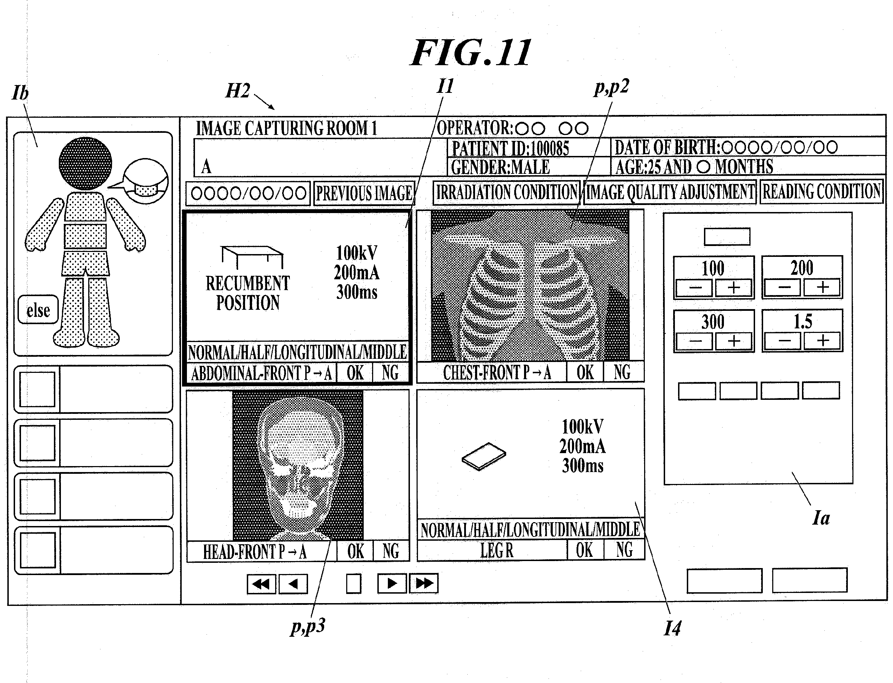

Then, by changing the radiation emission direction by changing the direction in which the radiation source 52 faces and changing the X-ray tube current and the like, the radiographic image capturing specified in the radiation capturing order information having the image capturing order ID "001" of FIG. 7 can be performed in a state where minimum changing is required by using the radiation source 52 which is already activated. Therefore, the console 58 switches the focus-display to the icon I1 as shown in FIG. 11.

Then, because the CR cassette 1C having the cassette ID "CR-003" is specified in the image capturing order information corresponding to the icon I1 (that is, the image capturing order information having the image capturing order ID "001" in FIG. 7), the controlling method needs to be switches to the controlling method for CR apparatus.

Thus, in such case, the console 58 sends the signal indicating that radiographic image capturing is to be performed by using CR apparatus to the radiation emission device 57. When the radiation emission device 57 receives the above signal from the console 58, the radiation emission device 57 switches the controlling mode to the mode for performing radiographic image capturing by using CR apparatus.

If the emission start signal is sent to the radiation emission device 57 from the exposure switch 56 by an operator such as a technician operating the exposure switch 56, at that time, the radiation emission device 57 is to be in a state to make the radiation source 52 emit radiation immediately in response to the emission start signal.

However, in the case where the signal indicating that radiographic image capturing is to be performed by using CR apparatus is sent to the radiation emission device 57 from the console 58 as described above, if it is configured that the console 58 automatically sends the signal, there is a possibility that an operator such as an technician does not notice that the controlling method was switched and that the radiographic image capturing is to be performed by using CR apparatus.

If such situation occurs, there is a possibility that an operator mistakes that it is image capturing using FPD apparatus and performs radiographic image capturing for a plurality of times by continuously emitting radiation to the CR apparatus without performing reading processing of image data D from a stimulable phosphor plate. Further, if image capturing is continuously performed for a plurality of times on a stimulable phosphor plate of CR apparatus, the image data D to be read out thereafter will be in a state where the plurality of times of image capturing are overlapped and such image data D is useless (not available for diagnosis).

Therefore, the plurality of times of radiographic image capturing is wasted and recapturing needs to be performed. This can lead to a problem such that total exposure dose of the patent increases and the burden on the patient becomes extremely large.

In view of the above, in the embodiment, the console 58 makes a display indicating that radiographic image capturing is to be performed by using CR apparatus be displayed in the display unit 58a before the signal indicating that radiographic image capturing is to be performed by using CR apparatus is sent to the radiation emission device 57 so that an operator will accurately confirm that the radiographic image capturing is image capturing performed by using CR apparatus (that is, that the image capturing method is the CR method). Only when an operator agrees with such display, the consol 58 sends the above signal to the radiation emission device 57.

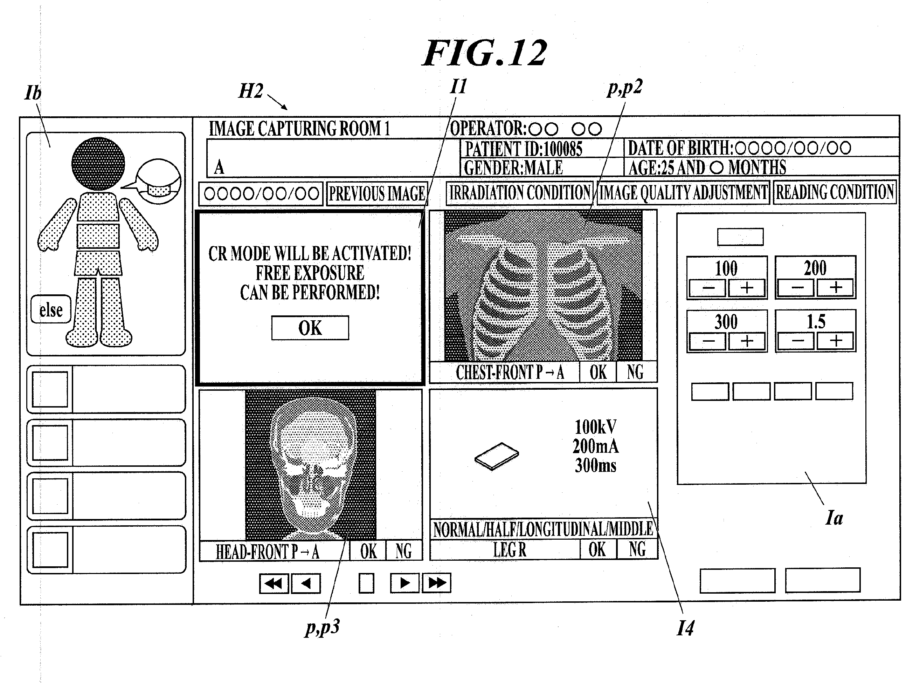

In particular, when the console 58 switches the focus-display to the icon I1 corresponding to the image capturing order information which specifies CR apparatus as shown in FIG. 11, the console 58 displays a display indicating that radiographic image capturing is to be performed by using CR apparatus in the icon I1 section with "OK" button icon as shown in FIG. 12, for example.

The configuration may be such that only when it is agreed to perform the radiographic image capturing apparatus by using CR apparatus by an operator clicking the "OK" button Icon, the console 58 sends the signal indicating that radiographic image capturing is to be performed by using CR apparatus to the radiation emission device 57.

Further, although omitted in the drawings, configuration may be such that the above display is displayed in the screen H2, full screen thereof, so that an operator cannot perform other operations in the screen H2 unless the operator clicks the "OK" button icon in order to make the operator unfailingly confirm that the next image capturing is radiographic image capturing using CR apparatus.

By having such configuration, an operator can be made to unfailingly confirm that the next image capturing is radiographic image capturing using CR apparatus by the console 58 making the display unit 58a display the display indicating that the radiographic image capturing is to be performed by using CR apparatus and by allowing a signal indicating that radiographic image capturing is to be performed by using CR apparatus be sent to the radiation emission device 57 only when the above display was confirmed.

Therefore, performing multiple image capturing on the stimulable phosphor plate of the CR apparatus can be prevented appropriately and the exposure dose of a patient can be appropriately prevented from meaninglessly increasing.

Here, in the above, a case where the above mentioned display is displayed in the icon I section (in the icon I1 section in the example shown in FIG. 12) corresponding to the next image capturing in order to make an operator unfailingly recognize that the next image capturing is radiographic image capturing using CR apparatus is described.

Although omitted in the drawings, configuration may be such that a similar display is displayed at edge sections in various types of screens on the display unit 58a of the console 58 by which an operator performs operations so that an operator can switch the controlling mode of the system to the mode for performing radiographic image capturing using CR apparatus at any time.

That is, the console 58 makes a display indicating that the mode can be switched to the mode for performing radiographic image capturing using CR apparatus be displayed in various types of screens (not only in the image capturing screen, but also in the order list screen, maintenance screen, system setting screen and the like) on the display unit 58a and the signal indicating that radiographic image capturing is to be performed by using CR apparatus is to be sent to the radiation emission device 57 when an operator confirms the above display.

By having such configuration, an operator can switch the controlling mode of the system to the mode for performing radiographic image capturing using CR apparatus at any time. At that time, configuration may be such that the similar display is removed from each screen after the controlling mode of the system is switched to the mode for performing radiographic image capturing using CR apparatus. Further, configuration may be such that instead of the above mentioned display, a display indicating that the mode can be changed to the mode for radiographic image capturing using FPD apparatus is displayed in each screen and an operator can switch the controlling mode of the system to the mode for performing radiographic image capturing using FPD apparatus at any time.

On the other hand, once an operator such as a technician switches the controlling mode of the system to the mode for performing radiographic image capturing using CR apparatus, the operator knows that the controlling mode of the system is in the state for performing radiographic image capturing by using CR apparatus.

Therefore, in the embodiment, after the controlling mode of the system is switched to the mode for performing radiographic image capturing using CR apparatus, the above mentioned display (see FIG. 12) will not be displayed in a case where the focus-display of an icon I is to be switched in the screen H2 and the radiographic image capturing corresponding to the icon I to which the focus-display was switched is radiographic image capturing using CR apparatus.

When the radiographic image capturing corresponding to the icon I to which the focus display was switched is radiographic image capturing using FPD apparatus, the console 58 notifies an operator by displaying a display indicating that the controlling mode is switched to the mode for performing radiographic image capturing using FPD apparatus in the screen H2, for example, and sends a signal indicating that radiographic image capturing is to be performing by using FPD apparatus to the radiation emission device 57.

Until the above signal is sent from the console 58, the radiation emission device 57 remains to be in a state ready for making the radiation source 52 emit radiation immediately in response to an instruction when the instruction to start radiation emission is received from the exposure switch 56. However, the radiation emission device 57 switches the controlling mode to the mode for performing radiographic image capturing by using FPD apparatus when the above signal is sent from the console 58.

By having such configuration, the controlling method in the radiation emission device 57 can be appropriately switched at the time when switching the image capturing method (that is, either the CR method or the FPD method).

As described above, according to the radiographic image capturing system 50 of the embodiment, the controlling mode is set to the controlling method for FPD apparatus in the default state, and the controlling method in the radiation emission device 57 is switched in response to the signal indicating that radiographic image capturing is to be performing by using CR apparatus being sent to the radiation emission device 57 from the console 58, the signal being the trigger.

Further, in the default state where the above signal is not received, the radiation emission device 57 makes the radiation source 52 emit radiation for the first time when the interlock release signal is received directly from the FPD apparatus which is now in the condition for receiving radiation or via the console 58 in the way similar to the case where the radiation emission device 57 allows to emit radiation to the FPD apparatus when the emission start signal is received from the exposure switch 56.

Therefore, even when an operator such as a technician carelessly or unintentionally tries to emit radiation, radiation is to be emitted to the FPD apparatus only after the FPD apparatus is finished with the necessary processing such as reset processing of each radiation detection element 7 and is in the condition for receiving radiation. Thus, a problem such as exposure dose of a patient increasing due to careless radiation emission of an operator and the burden on the patient increasing can be appropriately prevented.

Moreover, the radiation emission device 57 switches the way of emitting radiation when the above mentioned signal is received. Further, similarly to the case where the radiation emission device 57 allows radiation to be emitted to the CR apparatus when the emission start signal from the exposure switch 56 is received, the radiation emission device 57 makes the radiation source 52 immediately emit radiation when the emission start signal from the exposure switch 56 is received.

Therefore, it can be appropriately prevented from not being able to emit radiation from the radiation source 52 forever due to the radiation emission device 57 waiting for the interlock release signal to be sent from the CR apparatus even though radiographic image capturing is to be performed by using CR apparatus, and radiographic image capturing to the CR apparatus can be performed appropriately.

According to the radiographic image capturing system 50 of the embodiment, in such way, careless or unintentional radiation emission by an operator such as a technician can be appropriately prevented and the controlling method can be switched appropriately at the time when switching the image capturing method in the radiographic image capturing system 50 including both the CR apparatus and the FPD apparatus.

Here, in the embodiment, the case where an operator such as a technician confirms the radiographic image p for diagnosis (see FIG. 10, for example) which is generated by the console 58 and clicks the "OK" button icon, and at this time, the image capturing is completed by the console 58 confirming the radiographic image p for diagnosis and making the radiographic image p be associated with the image capturing order information is described. However, there are requests for making various types of information relating to the image capturing, not only the radiographic image p which is the result of image capturing, be associated with the corresponding image capturing order information and be stored in the medical field.

In view of the above requests, in the embodiment, the radiation emission device 57 (see FIG. 1) sends image capturing performance results, which are, performance results relating to the X-ray tube current, X-ray tube voltage, irradiation time period and the like of when radiation is emitted to the console 58 every time radiation is emitted from the radiation source 52. Further, the console 58 makes the sent image capturing performance results be associated with the corresponding image capturing order information.

Moreover, configuration may be such that the area exposure product of radiation emitted to the FPD cassette 1F or the CR cassette 1C from the radiation source 52 in the radiographic image capturing, that is, the product of radiation area and exposure dose of radiation with respect to the cassette is also made to be associated with the corresponding image capturing order information.

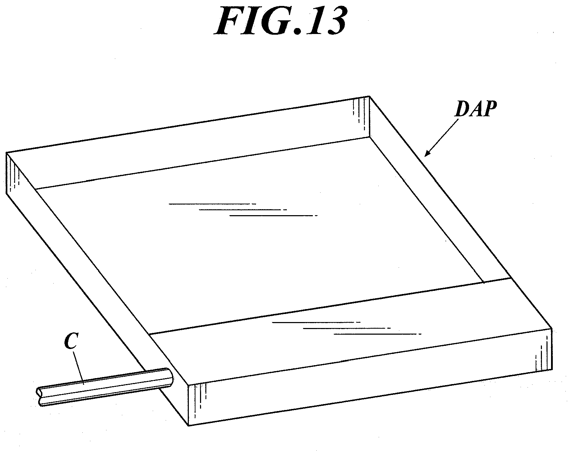

In such case, the FPD cassette 1F or the CR cassette 1C are fitted in an area exposure product meter DAP (also called, DAP (dose-area product) meter) to be loaded in the bucky 51 as shown in FIG. 13, for example. Alternatively, the FPD cassette 1F or the CR cassette 1C can be used by being fitted in the area exposure product meter DAP even in a case where the cassette is to be used in an alone sate not being loaded in the bucky 51.

When the area exposure product meter DAP is to be used along with the FPD cassette, the console can recognize that the radiographic image capturing is finished at the time when a preview image is sent from the FPD cassette. Therefore, the console 58 prepares for making associate between the image and the measurement result (on the other hand, when the area exposure product meter DAP is used along with the CR cassette, the console will not be able to recognize the finishing of radiographic image capturing in a timely basis).

Moreover, in the embodiment, the area exposure product meter DAP can perform sending and receiving signals, data and the like with the relay device 54 (see FIG. 1) in a wired manner via the cable C or in a wireless manner via an antenna device (not shown in the drawing).

Further, at the time when the radiographic image capturing is finished and sending of the entire data including image data D from the FPD cassette 1F is completed, the console 58 requests data to be sent from the area exposure product meter DAP in which the FPD cassette 1F used in the radiographic image capturing is fitted. Here, sending of the measurement result is requested after the sending of the necessary image data is finished in order to prioritize the sending of the obtained image data D and the decision whether diagnosis can be provided or not by an operator such as a technician (determination whether recapturing is needed or not). It is needless to say that the data can be requested to be sent from the area exposure product meter DAP immediately after the preview is received.

When sending of data is requested by the console 58, in response, the area exposure product meter DAP sends the area exposure product data measured at the time of the radiographic image capturing to the console 58. When the area exposure product data measured at the time of the radiographic image capturing is sent from the area exposure product meter DAP, the console 58 makes the received area exposure product data be associated with the image capturing order information corresponding to the radiographic image capturing.

Here, when recapturing of the image is needed because an operator who reviewed the preview image p_pre (see FIG. 9) relating to the radiographic image capturing clicked the "NG" button icon or the radiographic image p for diagnosis (see FIG. 10) generated on the basis of the image data D and the like obtained in the radiographic image capturing is not approved by an operator, the console 58 sends a signal to instruct resetting (that is, deleting) the area exposure product data measured in the image capturing to the area exposure product meter DAP.

Moreover, in a case where a plurality of area exposure product meters DAP exist in the radiographic image capturing system 50, in the embodiment, configuration may be such that the identification information of an area exposure product meter DAP is sent to the console 58 by reading the barcode (not shown in the drawing) of the area exposure product meter DAP with the reading device (not shown in the drawing) provided in the cradle 55, for example, at the same time when registering the cassette ID of the FPD cassette 1F which is brought in the image capturing room Ra by inserting the cassette in the cradle 55 as described above so that the console 58 can specify the area exposure product meter DAP used in the radiographic image capturing.

Then, for example, the console 58 may make the cassette ID and the identification information of the area exposure product meter DAP be associated to each other to manage them thereafter until the release operation is performed.

In such case, for example, when a plurality of image capturing rooms Ra (for example, image capturing rooms Ra1 to Ra3) exist in the radiographic image capturing system 50 as shown in FIG. 14, by inserting the FPD cassette 1F in the cradle 55 in the image capturing room Ra to which the FPD cassette 1F was brought in to register the FPD cassette 1F every time when the FPD cassette 1F is brought into an image capturing room Ra, the console 58 can determine that the area exposure product meter DAP which is associated to the cassette ID of the FPD cassette 1F is also brought in to the image capturing room Ra.

Further, configuration may be such that the cassette ID of the FPD cassette 1F is registered by using the cradle 55 in the image capturing room Ra the cassette was brought in and the identification information of the area exposure product meter DAP is sent to the console 58 by reading the barcode of the area exposure product meter DAP with the reading device provided in the cradle 55 every time when the FPD cassette 1F and the area exposure product meter DAP are moved between the image capturing rooms Ra.

In such way, not only image data D, data of radiographic image p for diagnosis and the like but also image capturing performance results relating to the radiographic image capturing sent from the radiation emission device 57, area exposure product information measured by the area exposure product meter DAP and the like can be made to be associated to the corresponding image capturing order information.

On the other hand, in a case where the radiographic image capturing system is a system including both the FPD apparatus and the CR apparatus, that is, a system where the FPD cassette 1F and the CR cassette 1C are used and the dedicated type radiographic image capturing apparatus and the CR apparatus in which a stimulable phosphor plate and a reading device are integrally formed are used as in the radiographic image capturing system 50 of the embodiment, image data D and the like obtained by using various types of image capturing apparatuses are mixed in the console 58.

However, in many cases, it is convenient for an operator such as a technician that the above mixed image data D and the like are managed by being individually categorized and not stored and managed in a mixed manner.

In view of the above, configuration may be such that when storing the image data D and the like by associating them respectively to the image capturing order information, the console 58 categorizes the data in individual folders created for the image capturing apparatuses (that is, for each of the FPD cassette 1F, the dedicated type radiographic image capturing apparatus, the CR cassette 1C, the CR apparatus in which a stimulable phosphor plate and a reading device are integrally formed and the like).

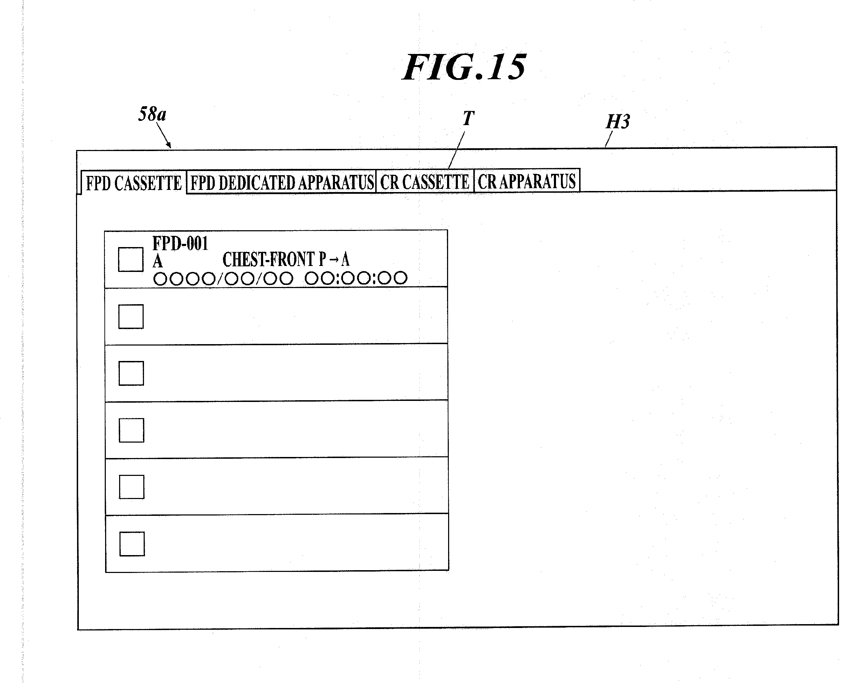

Also, configuration may be such that when the data is to be displayed in the display unit 58a of the console 58, a tab T is provided for each image capturing apparatus, that is, for each of the FPD cassette 1F, the dedicated type radiographic image capturing apparatus, the CR cassette 1C, the CR apparatus in which a stimulable phosphor plate and a reading device are integrally formed and the like in the screen H3 as shown in FIG. 15, and data stored in the folder of each image capturing apparatus is displayed by clicking each tab.

By having such configuration, the image data D and the like obtained by using each of the image capturing apparatuses can also be categorized by image capturing apparatus to be managed and stored in the radiographic image capturing system 50 including both the FPD apparatus and the CR apparatus. Therefore, it will be easier for an operator such as a technician to manage and analyze the image data D and the like and user-friendliness of the radiographic image capturing system 50 can be improved.

Specifically, with respect to the FPD cassette 1F and the dedicated type radiographic image capturing apparatus, that is, the FPD apparatus, quality of the FPD apparatus needs to be maintained by regularly performing quality control (so-called, QC) at the time of its installation in a facility and after its introduction.

In a case where the radiographic image capturing system including both the CR apparatus and the FPD apparatus is to be established by newly installing the FPD apparatus in a facility in which the CR apparatus is already installed as described above, it is preferred to perform quality control of the FPD apparatus by using the QC phantom for the CR apparatus.

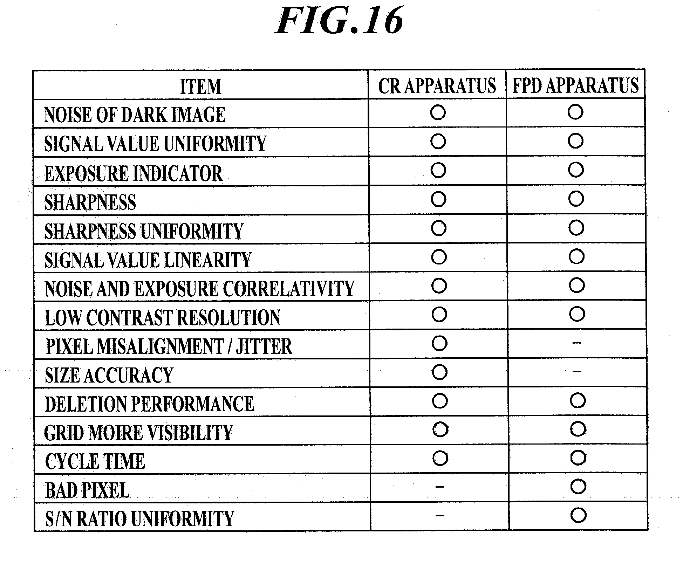

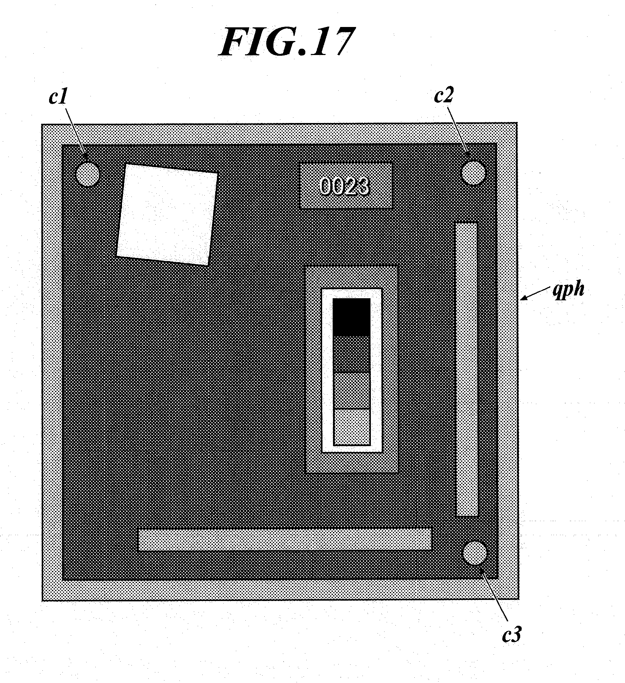

For example, when complying with the standard of AAPM (American Association of Physicists in Medicine), QC test is to be performed for items such as those indicated with ○ in FIG. 16, for example, with respect to the CR apparatus. As for the QC tool for performing the QC test specifically to the item "size accuracy", there is a case where the QC phantom qph as shown in FIG. 17, for example, is used.

In the QC phantom qph shown in FIG. 17, copper pieces c1 to c3 are respectively provided in three of the corners, for example, and radiation is emitted to the CR apparatus through the QC phantom qph and the spaces between the copper pieces c1 to c3 which are read as image data D by being captured on a stimulous phosphor plate, that is, the space in horizontal direction in the drawing is obtained from the space between the copper pieces c1 and c2 and the space in vertical direction in the drawing is obtained from the space between the copper pieces c2 and c3 to measure the size accuracy.

In a radiographic image capturing system in which the CR apparatus is already installed, such QC phantom already exists. Therefore, a case where the radiographic image capturing system including both the CR apparatus and the FPD apparatus is to be established by newly installing the FPD apparatus in a facility in which the CR apparatus is already installed as described above, quality control of the FPD apparatus can be performed by using the QC phantom qph for the CR apparatus.

Specifically, it is considered that quality control is to be performed for the items indicated with ○ in FIG. 16 with respect to the FPD apparatus. By using the QC phantom qph as shown in FIG. 17 which already exits in the system, measurement of "sharpness uniformity" with respect to the FPD apparatus can be performed, for example.

As for the indicator for sharpness, there is known a method using so-called response function (MTF), and a rectangle chart method, a slit method, an edge method and the like are known as the measuring methods of MTF.

When measuring of "sharpness uniformity" with respect to the FPD apparatus is performed by using the above methods, radiation is emitted to the FPD apparatus via the QC phantom qph and sharpness of each part is to be calculated in a numerical value by applying the above mentioned method to the image data D of the sections corresponding to the copper pieces c1 to c3 in the read image data D.

Further, in the numerical values indicating the sharpness of the sections corresponding to the copper pieces c1 to c3, the minimum value is subtracted from the maximum value and the obtained value is divided by the maximum value, for example. Thereby, the index RU of "sharpness uniformity" is obtained.

In such case, sharpness in the image data D in the sections corresponding to the copper pieces c1 to c3 show approximately the same numeral values when the sharpness uniformity is maintained, and therefore, the index RU of "sharpness uniformity" is to be a value near 0. Further, sharpness in the image data D in the sections corresponding to the copper pieces c1 to c3 will not be the same numeral values when the sharpness uniformity is not maintained, and therefore, the index RU of "sharpness uniformity" is to be significantly different from 0.

In such way, by calculating the index RU of "sharpness uniformity" as described above, how much the "sharpness uniformity" is maintained in the FPD apparatus can be determined from the calculated value RU.

As described above, in a case where the radiographic image capturing system including both the CR apparatus and the FPD apparatus is to be established by newly installing the FPD apparatus in a facility in which the CR apparatus is already installed, quality control of the FPD apparatus which to be newly installed can be appropriately performed by using the QC phantom qph for the CR apparatus which already exists in the system and the quality of the FPD apparatus can be maintained properly.

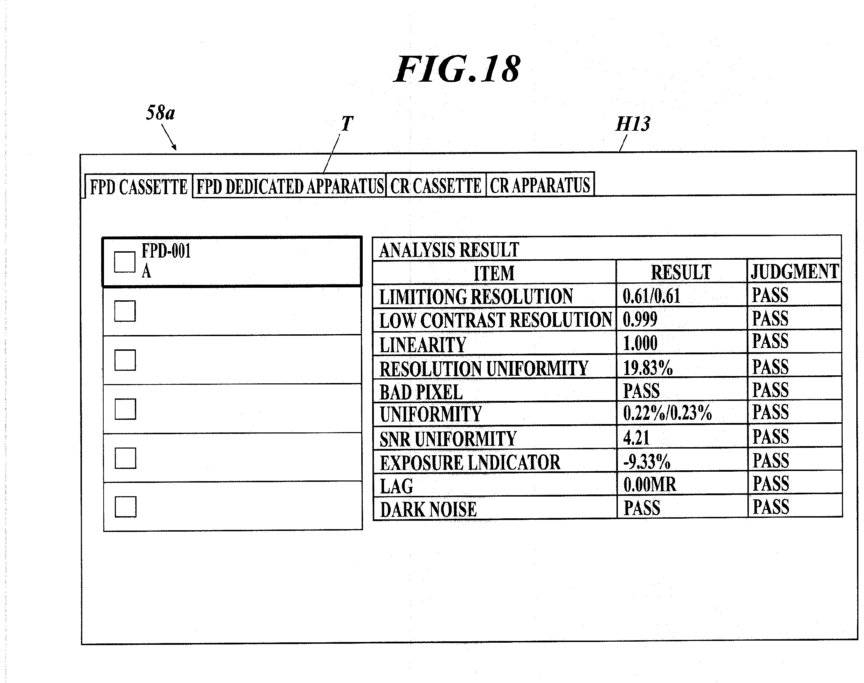

Moreover, as shown in FIG. 18, by generating phantom image capturing data of each type of image capturing apparatus (the FPD cassette, the FPD dedicated apparatus, the CR cassette, the CR apparatus) included in the facility in the same console at the time of introduction and regularly thereafter and storing the analyzed results (see right side in FIG. 18), performance transition in the image capturing apparatuses can be managed.

Furthermore, it is preferred that the analyzed results are stored with the phantom image capturing data and the phantom image capturing data is output and displayed with the analyzed results. Here, in FIG. 18, a case where the analyzed results for the FPD cassette 1F having cassette ID "FPD-0001" selected in the plurality of FPD cassette 1F is displayed is shown.

Here, it is needless to say that the present invention is not limited to the above described embodiment and modifications can be carried out arbitrarily within the scope of the present invention.

## Claims

1. A radiographic image capturing system (50) including;
a radiation emission device (57);
a FPD cassette (1F) which is configured to receive radiation emitted from the radiation emission device (57) and generate image data; and
a console (58) which is configured to store the image data sent from the FPD cassette (1F) so as to be associated with image capturing order information,
**characterized in that**
the radiographic image capturing system (50) includes an area exposure product meter (DAP), wherein said product is the product of radiation area and exposure dose of radiation with respect to the cassette, the area exposure product meter (DAP) is configured to send measured area exposure product data to the radiographic image capturing console (58), and
the console (58) is configured to store the area exposure product data so as to be associated with the image capturing order information.

2. The radiographic image capturing system (50) of claim 1, wherein the area exposure product meter (DAP) is configured to fit the FPD cassette (1F) therein.

3. The radiographic image capturing system (50) of claim 2, wherein
the radiographic image capturing system (50) includes a bucky (51), and
the area exposure product meter (DAP) is configured so as to be loaded in the bucky (51).

4. The radiographic image capturing system (50) of claims 1 to 3, wherein
the console is configured to request the area exposure product data to be sent from the area exposure product meter (DAP) after receiving the image data from the FPD cassette (1F), and
the area exposure product meter (DAP) is configured to send the area exposure product data to the console in response to the request from the console.

## Patentansprüche

1. Radiographisches Bilderfassungssystem (50), beinhaltend:
eine Strahlungsemissionsvorrichtung (57);
eine FPD-Kassette (1F), die konfiguriert ist, um von der Strahlungsemissionsvorrichtung (57) emittierte Strahlung zu empfangen und Bilddaten zu erzeugen; und
eine Konsole (58), die konfiguriert ist, um die von der FPD-Kassette (1F) gesendeten Bilddaten zu speichern, sodass sie mit Informationen zur Bilderfassungsreihenfolge assoziiert werden,
**dadurch gekennzeichnet, dass**
das radiographische Bilderfassungssystem (50) einen Bereichsexpositionsproduktmesser (DAP) beinhaltet, wobei das Produkt das Produkt von Strahlungsbereich und Expositionsdosis von Strahlung in Bezug auf die Kassette ist, wobei der Bereichsexpositionsproduktmesser (DAP) konfiguriert ist, um gemessene Bereichsexpositionsproduktdaten an die radiographische Bilderfassungskonsole (58) zu senden und die Konsole (58) konfiguriert ist, um die Bereichsexpositionsproduktdaten zu speichern, sodass sie mit den Informationen zur Bilderfassungsreihenfolge assoziiert werden.

2. Radiographisches Bilderfassungssystem (50) nach Anspruch 1, wobei der Bereichsexpositionsproduktmesser (DAP) konfiguriert ist, sodass die FPD-Kassette (1F) darin hineinpasst.

3. Radiographisches Bilderfassungssystem (50) nach Anspruch 2, wobei das radiographische Bilderfassungssystem (50) ein Bucky (51) beinhaltet und
der Bereichsexpositionsproduktmesser (DAP) konfiguriert ist, um in das Bucky (51) geladen zu werden.

4. Radiographisches Bilderfassungssystem (50) nach Anspruch 1 bis 3, wobei die Konsole konfiguriert ist, um nach dem Erhalt der Bilddaten von der FPD-Kassette (1F) die von dem Bereichsexpositionsproduktmesser (DAP) zu sendenden Bereichsexpositionsproduktdaten anzufordern und
der Bereichsexpositionsproduktmesser (DAP) konfiguriert ist, um die Bereichsexpositionsproduktdaten als Reaktion auf die Anforderung von der Konsole an die Konsole zu senden.

## Revendications

1. Système de capture d'image radiographique (50) incluant :
un appareil d'émission de rayonnement (57) ;
une cassette FPD (1F) qui est configurée pour recevoir un rayonnement émis depuis l'appareil d'émission de rayonnement (57) et générer des données image ; et
une console (58) qui est configurée pour stocker les données image envoyées depuis la cassette FPD (1F) de façon à ce qu'elles soient associées à des informations d'ordre de capture d'image, **caractérisé en ce que** le système de capture d'image radiographique (50) inclut un compteur de produit d'exposition de zone (DAP), dans lequel ledit produit est le produit de zone de rayonnement et la dose d'exposition de rayonnement par rapport à la cassette, le compteur de produit d'exposition de zone (DAP) est configuré pour envoyer des données de produit d'exposition de zone mesuré à la console de capture d'image radiographique (58), et la console (58) est configurée pour stocker les données de produit d'exposition de zone de façon à ce qu'elles soient associées aux informations d'ordre de capture d'image.

2. Système de capture d'image radiographique (50) selon la revendication 1, dans lequel le compteur de produit d'exposition de zone (DAP) est configuré pour loger la cassette FPD (1F) à l'intérieur de celui-ci.

3. Système de capture d'image radiographique (50) selon la revendication 2, dans lequel le système de capture d'image radiographique (50) inclut une grille antidiffusante (51), et le compteur de produit d'exposition de zone (DAP) est configuré de façon à être chargé dans la grille antidiffusante (51).

4. Système de capture d'image radiographique (50) selon les revendications 1 à 3, dans lequel la console est configurée pour demander l'envoi des données de produit d'exposition de zone depuis le compteur de produit d'exposition de zone (DAP) après réception des données image depuis la cassette FPD (1F), et le compteur de produit d'exposition de zone (DAP) est configuré pour envoyer les données de produit d'exposition de zone à la console en réponse à la demande de la console.
